Europäisches Patentamt

European Patent Office

Office européen des brevets

(11) Publication number: **0 328 870**
**A2**

(12) **EUROPEAN PATENT APPLICATION**

(21) Application number: **89100497.0**

(22) Date of filing: **12.01.89**

(51) Int. Cl.⁴: **A23K 1/18 , A23K 1/17 , A61K 37/36**

(30) Priority: **19.02.88 US 158020**

(43) Date of publication of application:
**23.08.89 Bulletin 89/34**

(84) Designated Contracting States:
**AT BE DE ES FR GB GR IT LU NL SE**

(71) Applicant: **AMERICAN CYANAMID COMPANY**
**1937 West Main Street P.O. Box 60**
**Stamford Connecticut 06904-0060(US)**

(72) Inventor: **Simkins, Karl LeRoy, Jr.**
**4 Wellington Drive**
**Princeton Junction New Jersey 08550(US)**
Inventor: **Rock, David William**
**50 Montgomery Road**
**Neshawic Station Jersey 08855(US)**

(74) Representative: **Wächtershäuser, Günter, Dr.**
**Tal 29**
**D-8000 München 2(DE)**

(54) Improvement in milk production from lactating ruminants while increasing milk fat and lactose content in the milk produced.

(57) There is provided a method for enhancing the production of milk by lactating ruminants and increasing the milk fat and lactose content of the milk produced, by orally administering to lactating ruminants from about 50 to 150 mg per head per day of a glycopeptide antibiotic or polyether ionophore and in conjunction therewith, parenterally administering to said ruminants from about 5 to 50 mg per head per day of bovine somatotropin.

EP 0 328 870 A2

# IMPROVEMENTS IN MILK PRODUCTION FROM LACTATING RUMINANTS WHILE INCREASING MILK FAT AND LACTOSE CONTENT IN THE MILK PRODUCED

## SUMMARY OF THE INVENTION

The present invention relates to a method for improving milk production in lactating animals and increasing the milk fat and lactose content of the milk produced. These desirable objectives can be achieved through the parenteral administration of about 5 to 50 mg/head/day of bovine somatotropin (BST) to each of the lactating ruminants and in conjunction therewith, orally administering to each of said ruminants about 50 to 150 mg/head/day of a glycopeptide antibiotic or a polyether ionophore.

## DETAILED DESCRIPTION OF THE PREFERRED EMBODIMENTS

The BST may be administered on a daily basis, in the form of a dilute solution, via subcutaneous injection or on a continuous basis in the form of a slow release implant of injectable formulation with sustained-release properties. when the latter form of administration is used the sustained release formulation is prepared in such manner as to release only about 5 to 50 mg/head/day of BST into the body fluids of the treated animals.

BST compositions useful in the practice of this invention are generally comprised of soluble carbohydrate polymer to growth hormone ratios on a weight basis of from about 1.0/1.0 to 100.0/1.0. Soluble carbohydrate polymer to solvent ratio will of course vary depending upon the solubility of the carbohydrate and solvent employed. The compositions having soluble carbohydrate polymer to growth hormone ratios of from about 1.0/1.0 to 100/1 may be administered as aqueous pastes or solutions in water or buffered saline solutions. Higher ratios of water soluble polymer may also be employed. The quantity of water or buffered saline solution can vary depending upon the carbohydrate polymer and its solubility characteristics, as may the pH of the buffered saline solution. Aqueous compositions of the invention administered to animals in a pH range of from about pH 3.0 to pH 10.0 having water to carbohydrate polymer ratios of about 0.83 to 1 to 7.5 to 1 are effective for administering and maintaining increased levels of bovine growth hormone in animals.

Polymers useful in the preparation of the bovine growth hormone compositions include: carbohydrate polymers such as the dextrans, dextrins, alginates, starches and fractionated starches, glycogen, pullullan, agarose, cellulose, chitosan, carrageenan, and synthetic biopolymers, as well as gums such as xanthan gum, guar gum, locust bean gum, gum arabic, tragacanth gum, and kara gum, derivatives thereof and mixtures thereof.

Solvents suitable for use in the bovine growth hormone compositions useful in the practice of this invention include phosphate buffered saline (PBS) which contains $NaH_2PO_4.H_2O$ (0.025 mol), $Na_2HPO_4$ - (0.025 mol), and NaCl (0.15 mol) which has been adjusted to pH 7.1; and Carbonate Buffer Saline (CBS) which contains $Na_2CO_3$ (0.025 mol), $NaHCO_3$ (0.025 mol), and NaCl (0.15 mol) which has been adjusted to pH 9.4; and saline; alone in combination with other pharmaceutically and pharmacologically acceptable water miscible solvents.

Pharmaceutically and pharmacologically acceptable solvents which may also be employed in biological composition for parenteral administrations include a variety of oils, waxes, etc., liquid alcohols, glycols, esters and amides.

A typical preparation of an aqueous bovine growth hormone composition useful in the practice of this invention can be accomplished as follows:

A low viscosity corn dextrin (18 g), having the properties of being acidic as a 25% dispersion with a viscosity of 50 centipoise at 25° C, and bovine growth hormone (0.18 g) is admixed with 15 mL of carbonate buffer saline, pH 9.4 ($Na_2CO_3$, 0.025 mol; $NaHCO_3$ 0.025 mol; and NaCl 0.15 mol) until a homogeneous paste is obtained.

Slow release pellets containing bovine growth hormone may be prepared by admixing about 85% to 95% by weight of the bovine growth hormone with about 5% to 15% by weight of an inert diluent such as carnuba wax, carbowax or the like. Other excepients such as corn starch acacia or the like may also be added if desired and a lubricant such as calcium or magnesium stearate may be incorporated into the mixture to aid in the manufacture of the pellets. In practice, it has been found that several pellets may be administered to each animal to achieve the desired daily release rate of BST of from 5 to 50 mg/head/day. It is also found that additional pellets may be administered to the animals at intervals during the lactation period to maintain the desired drug level in the animals.

A particularly useful parenteral composition effective for use in this invention comprises about 20% to 40% by weight of bovine growth hormone; about 55% to 790% glyceryl tristearate; 0 to about 0.20% microbiological preservative; 0 to about 10% non-ionic surfactant; and 0 to about 20% sodium benzoate.

In accordance with the present invention, it is also essential to provide each of the lactating animals with an effective amount of an orally administered glycopeptide antibiotic or polyether ionophore, such as avoparcin, actaplanin, ristocetin, vancomycin, lincomycin, virginiamycin, carbodox, monesin, lasolacid or bambermycin.

Avoparcin, also referred to as AV 290, is a preferred glycopeptide for use in this invention and may be employed in the variety of forms including AV 290, as described in United States Patent No. 3,338,786; AV 290 sulfate disclosed in United States Patent No. 3,855,410; AV 290 - syntan complex, described in United States Patent No. 3,832,462; AV 290 - alkyl sulfate complex disclosed in United States Patent No. 3,856,937; or an AV 290 alkylated derivative such as described in United States Patent No. 3,954,973. Although any of the above mentioned derivatives or complexes of AV 290 can be used in the treatment of lactating ruminants by the method of the present invention, AV 290 sodium lauryl sulfate is especially preferred.

Oral administration of the glycopeptide antibiotic or polyether ionophore can, advantageously, be achieved by uniformly dispersing the desired glycopeptide or polyether ionophore or a pharmaceutically acceptable salt, complex or derivative thereof in the animal feed and providing the thus prepared medicated feed to the animals. The polyether ionophore or glycopeptide may also be formulated for oral administration as feed concentrates, premixes, tablets or boluses, which may be prepared by accepted and well known methods, using pharmaceutically acceptable carriers, diluents, lubricating agents and binders.

An avoparcin bolus which is especially effective for use in the present invention can be prepared by admixing avoparcin 10% to 180%, preferably as AV 290 sodium lauryl sulfate; 100% to 180% montan acid wax; 100% to 18% carnuba wax; and 520% to 660% borite. The borite to total wax weight ratios being 1.4/1 to 3/1. The mixture is blended in equipment maintained at 80° C for ten minutes.

After blending the mixture is discharged and transferred to a pelletizing extruder and/or directly charged to an injection molder.

In the case of direct feeding of the dry blend, a feeding system such as a Vibra-Screw may be used, to transfer the material to an injection molding system utilizing a hot runner system and a shut-off nozzle. Operating conditions for the injection molding process includes feeding the blend into the screw at a temperature below 60° C, gradually heating the blend to a temperature above its melting point and forcing the molten blend into the mold. The mold is cooled to solidify the bolus and the bolus is removed weighing about 50 grams each having a density of about 2.4 g/cc and a hardness as measured on a Delamar Press of about 75 kg.

If pelletizing is carried out before the injection molding, the blended mixture is pelletized using a bell shaped temperature profile that is a cooled feeding section (50° -60° C), hot transition section (100° -110° C) and a cooled metering section (5° -60° C) with a straight-through 1/8 inch die equipped with a 20 mesh screen breaker plate. The die temperature is maintained at 55° -60° C. Resulting extruded rope is air cooled (air stream blowing at the die opening) and cut using a hot face cutter to 1/4" in long pellets.

It is understood that the technology of increasing lactation in lactating ruminants, improving the milk fat and lactose concentrations of milk from lactating ruminants and increasing the butter fat and lactose concentrations in milk produced by lactating ruminants encompasses parenterally administering bovine somatotropin, while concurrently administering orally to said lactating ruminants a tetracycline-type antibiotic or a macrolid antibiotic. The tetracycline-type antibiotics include the parent compound, tetracycline, and derivatives such as chlortetracyclin, 5-oxytetracycline, 5-chlortetracycline and 7-oxytetracycline. The macrolid antibiotics include tylosin.

EXAMPLE 1

Evaluation of the effects of bovine somatotropin (BST) and avoparcin concurrently administered to lactating animals.

In this trial forty-eight dairy cows are housed indoors in cubicles and offered grass silage ad libitum along with supplementary concentrates. All animals are offered similar nutrition levels on a daily basis. The concentrate feeding regimen is based on the estimated milk yield from the group of cows showing the highest production.

Animals are assigned into twelve groups, four cows per group, on the basis of (1) calving, (2) current lactation milk yield to 35 days post portum, (3) lactation number, (4) previous lactation milk yield, (5) milk coliform mastitis test and (6) condition score.

Treatments are allocated at random to animals within groups, as close to 42 days after calving as possible given the constraints of grouping. Treatments are as follows:

(a) control;

(b) 100 mg/day avoparcin (AVOTAN, 50) distributed in 1 kg dairy concentrates administered orally;

(c) 25 mg/day bovine somatotropin in 2 mL buffer administered as a sub-cutaneous injection; and

(d) 25 mg/day bovine somatotropin and 100 mg/day avoparcin administered as in B and C.

All animals are milked twice a day and medication administered to said animals on a daily basis. Animals on treatments (a) and (b) are injected daily with placebos. The control animals in group (a) and the animals in group (c) that receive only bovine somatotropin are given 1 kg per day of an unmedicated feed concentrate similar to the concentrate used with avoparcin but free of said avoparcin. All treatments are given on an individual basis for 84 days.

Feed additive and treated concentrates are analyzed prior to the treatment phase of the experiment.

Milk yield is measured daily, Monday through Saturday at milking, on Monday, Wednesday and Friday in the evening and on Tuesday, Thursday and Saturday in the morning. These data are taken from three weeks post calving until treatment is terminated (i.e. approximately 126 days) and then once every two weeks from then until the end of the lactation.

Milk samples are collected Wednesday evening and Thursday morning from calving until treatment is terminated and then once every month until the end of the lactation period. Separate morning and evening samples as collected and analyzed for protein, fat and lactose content.

Milk samples are also collected monthly and analyzed for cell count.

Concentrate intake is recorded on a daily basis and concentrates and silage analyzed monthly.

Body weight and condition scores are recorded at two week intervals. Reproductive efficiency (heat dates, service dates, etc.), gestation length and subsequent calf weights are also recorded. Pregnancy diagnosis is assessed at regular intervals by rectal palpation.

Plasma of milk samples is collected three times per week during the treatment period and assayed for progesterone to determine the date of first oestrus and mean progesterone concentrations during the oestrous cycle.

Additionally, plasma samples are collected from six animals per treatment on weeks two and eleven of treatment at 0, 1, 2, 4, 8, 12, 16 and 24 hours post injection and assayed for somatotropin concentration. Samples are assayed to detect both endogenous and exogenous growth hormone.

Clinical examination of each cow will be carried out weekly until day 126, and monthly until the end of the lactation.

Data obtained are reported in Table I below.

TABLE I

| Evaluation of the effect of concurrently administering to lactating animals bovine somatotropin (BST) and avoparcin | | | | |
|---|---|---|---|---|
| | Unmedicated control | 25 mg/day BST via injection | 100 mg/day avoparcin oral | 100 mg/ day avoparcin oral + 25 mg day BST via injection | % Yield Increase or decrease over controls |
| No of cows | 12 | 12 | 12 | 12 | - |
| Pretreatment yield, kg/day | 25.4 | 25.5 | 25.8 | 25.3 | - |
| Milk yield, kg/day | | | | | |
| 0 - 6 weeks | 21.8 | 26.3 | 22.7 | 27.4 | 25.7 |
| 6 - 12 weeks | 18.5 | 22.7 | 19.7 | 23.4 | 26.5 |
| 0 - 12 weeks | 20.2 | 24.5 | 21.2 | 25.3 | 25.2 |
| Fat, % | 4.17 | 4.39 | 4.17 | 4.44 | 6.5 |
| Protein, % | 3.44 | 3.35 | 3.42 | 3.35 | -2.6 |
| Lactose, % | 4.44 | 4.61 | 4.61 | 4.72 | 6.3 |

EP 0 328 870 A2

## Claims

1. A method for increasing lactation in lactating ruminants having a developed rumen function and improving the milk fat and lactose concentrations of milk from said lactating ruminants, characterized by; parenterally administering to said ruminants from about 5 to 50 mg/head/day of bovine somatotropin and in conjunction therewith, orally administering to said ruminants from about 50 to 150 mg/head/day of a glycopeptide antibiotic or polyether ionophore.

2. A method according to claim 1 wherein the polyether ionophore or glycopeptide is selected from avoparcin, lasalocid, monesin, actaplanin, vancomycin, ristocetin, lincomycin, virginamycin, carbodox or bambermycin.

3. A method according to claim 1 wherein the ruminants are cattle.

4. A method according to claim 1 wherein the antibiotic is avoparcin.

5. A method according to claim 1 wherein bovine somatotropin is administered daily, by injection, at the dose level of 25 mg/head/day and the antibiotic is avoparcin and is orally administered at 100 mg.head/day.

6. A method according to claim 5 wherein the ruminants are cattle, the bovine somatotropin is injected into said cattle daily at the dose level of 25 mg/head/day and the antibiotic is avoparcin and is administered at the dose level of 100 mg/head/day.

7. A method for increasing the butter fat and lactose concentrations in milk produced by lactating ruminants having a developed rumen function, characterized by; parenterally administering to said lactating ruminants about 5 to 50 mg/head/day of bovine somatotropin, while concurrently administering orally to said lactating ruminants from 50 to 150 mg/head/day of a glycopeptide antibiotic or polyether ionophore.

8. A method according to claim 7 wherein the polyether ionophore or glycopeptide is selected from avoparcin, lasalocid, monesin, actaplanin, vancomycin, ristocetin, lincomycin, virginamycin, carbodox or bambermycin.

9. As method according to claim 7 wherein the ruminants are cattle.

10. A method according to claim 7 wherein the antibiotic is avoparcin.